(19) Europäisches Patentamt
European Patent Office
Office européen des brevets

(11) **EP 1 109 775 B1**

(12) # EUROPÄISCHE PATENTSCHRIFT

(45) Veröffentlichungstag und Bekanntmachung des Hinweises auf die Patenterteilung:
**23.04.2003 Patentblatt 2003/17**

(21) Anmeldenummer: **99944427.6**

(22) Anmeldetag: **18.08.1999**

(51) Int Cl.[7]: **C07C 69/003**, C08G 83/00, C09D 201/00, C09J 201/00

(86) Internationale Anmeldenummer:
**PCT/EP99/06042**

(87) Internationale Veröffentlichungsnummer:
**WO 00/014049 (16.03.2000 Gazette 2000/11)**

(54) **HYPERVERZWEIGTE VERBINDUNGEN MIT EINER TETRAFUNKTIONELLEN ZENTRALGRUPPE UND IHRE VERWENDUNG**

HYPERBRANCHED COMPOUNDS WITH A TETRAFUNCTIONAL CENTRAL GROUP AND USE OF SAME

COMPOSES HYPERRAMIFIES DOTES D'UN GROUPE CENTRAL TETRAFONCTIONNEL ET LEUR UTILISATION

(84) Benannte Vertragsstaaten:
**BE DE ES FR GB IT**

(30) Priorität: **05.09.1998 DE 19840605**

(43) Veröffentlichungstag der Anmeldung:
**27.06.2001 Patentblatt 2001/26**

(73) Patentinhaber: **BASF Coatings AG**
**48165 Münster (DE)**

(72) Erfinder:
- **RINK, Heinz-Peter**
  **D-48153 Münster (DE)**
- **MIKOLAJETZ, Dunja**
  **D-48163 Münster (DE)**

(74) Vertreter: **Fitzner, Uwe, Dr.**
**Dres. Fitzner & Münch**
**Rechts- und Patentanwälte**
**Lintorfer Strasse 10**
**40878 Ratingen (DE)**

(56) Entgegenhaltungen:
**EP-A- 0 743 335**
**WO-A-93/17060**
**WO-A-96/12754**
**US-A- 5 136 014**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindungen betrifft eine neue hyperverzweigte Verbindung mit einer tetrafunktionellen Zentralgruppe. Außerdem betrifft die vorliegende Erfindung ein neues Verfahren zur Herstellung hyperverzweigter Verbindungen. Desweiteren betrifft die vorliegende Erfindung die Verwendung der neuen hyperverzweigten Verbindung als funktionelle Komponente in Mehrstoffmischungen und für Herstellung von Dendrimeren höherer Generationen.

Hyperverzweigte Verbindungen und Dendrimere, welche eine tetrafunktionellen Zentralgruppe enthalten, sind aus der Patentschrift WO 93/17060 bekannt. Hierbei wird als Zentralgruppe ein Tetrol wie Pentaerythrit, Ditrimethylolpropan, Diglycerin und Ditrimethylolethan verwendet. Indes sind die entsprechenden hyperverzweigten Verbindungen für zahlreiche Verwendungszwecke zu viskos und zu schlecht löslich. So ist es beispielsweise nicht möglich, in wäßrigen oder organischen Medien gelöste oder dispergierte Beschichtungsmittel, Klebstoffe oder Dichtungsmassen mit besonders hohem Feststoffgehalt herzustellen, wenn die bekannten hyperverzweigten Verbindungen angewandt werden. Auch die flüssigen Beschichtungsmittel, Klebstoffe oder Dichtungsmassen, welche die bekannten hyperverzweigten Verbindungen als funktionelle Komponente enthalten, sind für die meisten Applikationtechniken zu viskos. Dementsprechend weisen Pulverlacke und Pulverslutries mit bekannten hyperverzweigten Verbindungen Filmbildungstemperaturen auf. welche häufig zu nahe bei der Vernetzungstemperatur liegen.

Die EP 0 743 335 beschreibt als hyperverzweigte Verbindungen Aspartatester, Zentralgruppen wie gemäß der erfindungsgemäß angegebenen Formel lassen sich nicht konstruieren. Ebenfalls wird in der EP 0 743 335 kein Zusammenhang zwischen der Symmetrie der hyperverzweigten Verbindungen und den physikalischen Eigenschaften hergestellt. Die US 3,156,014 beschreibt Polymere, die radikalisch verzweigt werden, eine der erfindungsgemäßen Formel entsprechende Verbindung läßt sich nicht konstruieren. Auch hier wird kein Zusammenhang zwischen der Symmetrie der hyperverzweigten Verbindungen und den physikalischen Eigenschaften hergestellt.

Aufgabe der vorliegenden Erfindung ist es, neue hyperverzweigte Verbindungen zu finden, welche die Nachteile des Standes der Technik nicht mehr aufweisen und es ermöglichen, neue Beschichtungsmittel. Klebstoffe und Dichtungsmassen herzustellen, welche selbst bei hohem Festkörpergehalt oder in der Schmelze vorteilhaft niedrige Viskositäten aufweisen. Außerdem ist es die Aufgabe der vorliegenden Erfindung, ein neues Verfahren zur Herstellung von hyperverzweigten Verbindungen zu finden, das es gestattet, Verbindungen dieser Art in besonders einfacher Weise zu gewinnen. Desweiteren ist es die Aufgabe der vorliegenden Erfindung, neue Verwendungszwecke für hyperverzweigte Verbindungen, insbesondere als funktionelle Komponente in Mehrstoffmischungen und für die Herstellung von Dendrimeren höherer Generationen, zu finden.

Demgemäß wurden die neuen hyperverzweigte Verbindungen mit einer tetrafunktionellen Zentralgruppe der allgemeine Formel I gefunden

$$C[-A_q-X-]_m[-A_r-X-]_n[-A_s-X-]_o[-A_t-X-]_p \quad \text{(I)},$$

worin die Indices und die Variablen die folgende Bedeutung haben:

m+n+o+p=4; mit
m = eine ganze Zahl von 1 bis 3 und
n, o und p = 0 oder eine ganze Zahl von 1 bis 3;

q, r, s und t = eine ganze Zahl von 1 bis 5, wobei q > r, s, t;

X = -O-, -S- oder -NH-;

A = $-CR_2-$; mit

R = -H, -F, -Cl, -Br, -CN, $-NO_2$, $C_1$-$C_3$-Alkyl- oder -Haloalkyl- oder $C_1$-$C_3$-Alkoxirest oder, für q, r, s und/oder t = mindestens 2 einen $C_2$-$C_4$-Alkandiyl- und/oder -Oxaalkandiylrest, welcher 2 bis 5 Kohlenstoffatome und/oder ein Sauerstoffatom -O-, welches 3 bis 5 Kohlenstoffatome des Restes -A-überbrückt.

Im folgenden werden die neuen hyperverzweigten Verbindungen mit einer tetrafunktionellen Zentralgruppe der allgemeinen Formel I der Kürze halber als "erfindungsgemäßer Verbindungen" bezeichnet.

Die tetrafunktionellen Zentralgruppen der allgemeinen Formel I werden im folgenden der Kürze halber als "Zentralgruppen I" bezeichnet.

Dementsprechend wird das neue Verfahren zur Herstellung der erfindungsgemäßen Verbindungen in folgenden als "erfindungsgemäßes Verfahren" bezeichnet.

Ebenso werden im folgenden die neuen Mehrstoffmischungen, welche die erfindungsgemäßen Verbindungen enthalten, als "erfindungsgemäßen Mehrstoffmischungen", insbesondere als "erfindungsgemäße Beschichtungsmittel", "erfindungsgemäße Klebstoffe" oder "erfindungsgemäße Dichtungsmassen", bezeichnet.

Die neuen Dendrimere höherer Generationen, welche unter Verwendung der erfindungsgemäßer Verbindungen herstellbar sind, werden im folgenden als "erfindungsgemäße Dendrimere" bezeichnet.

Im Hinblick auf den Stand der Technik ist es überraschend, daß die Aufgabe der vorliegenden Erfindung mit Hilfe der erfindungsgemäßen Verbindungen gelöst werden konnte. Insbesondere stand es nicht zu erwarten, daß die Variation der Zentralgruppe, welche im Stand der Technik auch als Initiatorgruppe bezeichnet wird, so weitreichende technische Folgen haben würde.

Der wesentliche Bestandteil der erfindungsgemäßer Verbindungen ist die Zentralgruppe I.

In der allgemeinen Formel I addieren sich die Indizes m, n, o und p auf 4. Der Index m ist stets größer als 0 und steht für eine ganze Zahl von 1 bis 3, insbesondere für 1.

Die Indizes n, o und p haben unter Beachtung der vorstehenden Randbedingung den Wert 0 oder stehen für eine ganze Zahl von 1 bis 3. Dies bedeutet, daß nicht jeder dieser Indizes den Wert 0 annehmen kann.

Erfindungsgemäß sind folgende Wertekombinationen der Indizes von Vorteil:

m = 1 und n, o, p = 1;
m = 1, n = 2, o, p = 1;
m = 1, n = 2, o = 1 und p = 0;
m = 1, n = 3, o, p = 0;

m = 2, n = 1, o = 1 und p = 0;
m = 2, n = 2 und o, p = 0;

m =3, n = 1 und 0, p =0.

Von diesen sind die Zahlenkombinationen von besonderem Vorteil, in denen m = 1.

In der allgemeinen Formel I bezeichnen die Indizes q, r, s und t ganze Zahlen von 1 bis 5. Hierbei ist der Index q stets größer als die Indizes r, s und t. Demnach hat der Index q einen Wert von mindestens 2.

Erfindungsgemäß sind folgende Zahlenkombinationen der Indizes von Vorteil:

q = 2, r, s und/oder t = 1;
q = 3, r, s und/oder t = 1 und/oder 2;
q = 4, r, s und/oder t = 1, 2 und/oder 3;
q = 5, r, s und/oder t = 1, 2, 3 und/oder 4.

Die Variable -X- in der allgemeinen Formel I bezeichnet zweibindige Sauerstoffatome -O- oder Schwefelatome -S- oder eine sekundäre Aminogruppe -NH-. Erfindungsgemäß ist es von Vorteil, wenn -X- für -O- steht.

Die Variable -A- in der Formel I bedeutet einen zweibindigen Rest $-CR_2-$.

Der Rest R steht hierin für Wasserstoffatome -H, Fluoratome -F, Chloratome -Cl, Bromatome -Br, Nitrilgruppen -CN, Nitrogruppen $-NO_2$, $C_1$-$C_3$-Alkyl- oder - Haloalkylgruppen oder $C_1$-$C_3$-Alkoxigruppen. Beispiele geeigneter Gruppen dieser Art sind Methyl-, Ethyl-, Propyl-, Trifluormethyl-,Trichlormethyl-, Perfluorethyl-, Perfluorpropyl-, Methoxi-, Ethoxi- oder Propoxigruppen.

Erfindungsgemäß von Vorteil sind Wasserstoffatome oder Methylgruppen, welche daher bevorzugt verwendet werden. Insbesondere werden Wasserstoffatome verwendet. Bei den erfindungsgemäß besonders bevorzugten Variablen -A- handelt es sich demnach um Methylengruppen.

Steht in der allgemeinen Formel I mindestens einer der Indices q, r, s und/oder t mindestens für die Zahl 2 kann der Rest R auch für einen $C_2$-$C_4$-Alkandiyl- und/oder Oxaalkandiylrest stehen, welcher 2 bis 5 Kohlenstoffatome des Restes -A- cyclisch überbrückt. Der Rest -R- kann indes auch für ein Sauerstoffatom -O- stehen, welches 3 bis 5 Kohlenstoffatome des Restes -A- cyclisch überbrückt. Hierdurch werden Cyclopentan-1,2- oder -1,3-diyl-Gruppen, Tetrahydrofuran-2,3-, -2,4-, -2,5-oder -3,4-diyl-Gruppen, Cyclohexan-1,2-, -1,3- oder -1,4-diyl-Gruppen oder Tetrahydropyran-2,3-, 2,4-, 2,5- oder 2,6-diylgruppen, indes keine Epoxidgruppen, gebildet.

Beispiele erfindungsgemäß ganz besonders vorteilhafter Zentralgruppen I leiten sich demnach von den nachstehend beschriebenen Tetrolen (III1) bis (III10) formal ab, indem man die vier Wasserstoffatome der Hydroxylgruppen abstrahiert.

Von diesen ist die Zentralgruppe I, welche sich von dem Tretrol (III1) (2,2-Bishydroxymethyl-butandiol-(1,4);

Homopentaerythrit) ableitet, ganz besonders vorteilhaft und wird deshalb erfindungsgemäß ganz besonders bevorzugt verwendet.

In den erfindungsgemäßen Verbindungen sind die vorstehend beschriebenen Variablen -X- über Abstandshaltergruppen mit jeweils einer reaktiven funktionellen Gruppe verbunden. Im Rahmen der vorliegenden Erfindung ist unter den Begriff "reaktive funktionelle Gruppe" eine Gruppe zu verstehen, welche im Gegensatz zu einer inerten Gruppe für weitere Reaktionen leicht zur Verfügung steht. Demgemäß kann es sich um beliebige, von der organischen Chemie her bekannte Gruppen handeln. Erfindungsgemäß ist es indes von Vorteil, wenn es sich um eine Gruppe der allgemeinen Formel II handelt.

$$\textbf{-X-B} \qquad \text{(II)}$$

In der allgemeinen Formel II steht die Variable -X- für zweibindige Sauerstoff- und Schwefelatome oder für sekundäre Aminogruppen. Erfindungsgemäß ist es von Vorteile, wenn die Variable -X- für zweibindige Sauerstoffatome steht.

In der allgemeinen Formel II steht die Variable B für ein Wasserstoffatom -H, eine Gruppe -Z oder eine lineare oder verzweigte Gruppe $-R^1-(-Z^1)_u$, worin der Index u = 1 oder 2 ist.

Als Gruppen -Z kommen alle reaktiven funktionellen Gruppen der organischen Chemie in Betracht, welche unter den üblichen und bekannten Bedingungen Reaktionen eingehen, welche zu dem gewünschten weiteren Aufbau der erfindungsgemäßen Verbindung oder zu ihrer Verknüpfung mit anderen Verbindungen, wie etwa mit den von den Beschichtungsmitteln oder den Klebstoffen her bekannten Vernetzern. Beispiele für erfindungsgemäß besonders vorteilhafte Gruppen -Z sind:

$-C(O)-NH_2$,
$-C(O)-NH-C(O)-NH_2$,
$-C(O)-OR^2$,
$-C(O)-NH-C(O)-OR^2$,
$-C(O)-CH_2-C(O)-R^2$
$-C(O)-CH_2-C(O)-OR^2$
$-C(O)-CR^3 = CH_2$,

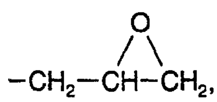

$-Si-(OR^4)_3$,
$-(-CH_2-)_v-CH = CH_2$ und
$-C(O)-(-CH_2-)_v-CH = CH_2$; worin der Index v gleich 0 ist oder für eine ganze Zahl von 1 bis 3;
$-C(O)-CH=CH-C(O)-O-R^2$,
$-C(O)-CH=CH-CH=CH-R^2$ und
$-C(O)-CH=CH-CH_2-CH=CH-R^2$.

Hierin bezeichnen die Reste $R^2$ gegebenenfalls substituierter $C_1$-$C_{10}$-Alkyl-, $C_5$-$C_{10}$-Cycloalkyl-, $C_6$-$C_{20}$-Cycloalkylalkyl-, $C_6$-$C_{20}$-Alkylcycloalkyl-, $C_6$-$C_{10}$-Aryl-, $C_7$-$C_{20}$-Arylalkyl-, $C_7$-$C_{20}$-Alkylaryl-, $C_{11}$-$C_{26}$-Arylcycloalkyl- oder $C_{11}$-$C_{26}$-Cycloalkylarylrest, wobei die Alkylreste verzweigt oder unverzweigt sind.

Als Substitutienten für diese Reste $R^2$ kommen alle organischen Reste in Betracht, welche im wesentlichen inert sind, d. h., daß sie keine Reaktionen mit den Verbindungen eingehen, welche für den Aufbau der erfindungsgemäßen Verbindungen oder für deren weitere Umsetzung verwendet werden. Beispiele geeigneter inerter organischer Reste sind Halogenatome, Nitrogruppen, Nitrilgruppen oder Alkoxygruppen.

Beispiele geeigneter erfindungsgemäß zu verwendender, gegebenenfalls substituierter $C_1$-$C_{10}$-Alkylreste sind Methyl-, Ethyl-, Propyl-, Butyl-, Pentyl-, Hexyl-, Heptyl-, Octyl-, Nonyl- oder Decylreste, von denen die Methyl-, Ethyl- und die Propylreste besonders vorteilhaft sind und deshalb bevorzugt verwendet werden.

Beispiele geeigneter erfindungsgemäß zu verwendender, gegebenenfalls substituierter $C_5$-$C_{10}$-Cycloalkylreste sind Cyclopentyl-, Cyclohexyl-, Cycloheptyl-, Cyclooctyloder Decalinylreste, von denen die Cyclopentyl- und Cyclohexylreste vorteilhaft sind und deshalb bevorzugt verwendet werden.

Beispiele geeigneter erfindungsgemäß zu verwendender, gegebenenfalls substituierter $C_6$-$C_{20}$-Cycloalkylal-

kylreste sind Cyclopentylmethyl-, Cyclopentylethyl-, Cyclohexylmethyl-, Cyclohexylethyl- oder Cyclohexylpropylreste, von denen der Cyclohexylmethylrest vorteilhaft ist und deshalb bevorzugt verwendet wird.

Beispiele geeigneter erfindungsgemäß zu verwendender, gegebenenfalls substituierter $C_6$-$C_{20}$-Alkylcycloalkylreste sind 4-Methyl-cyclohex-1-yl-, 2-Ethyl-cyclohex-1-yloder 4- Methyl-2-ethyl-cyclohex-1-yl-, von denen der 4-Methyl-cyclohex-1-yl-Rest vorteilhaft ist und deshalb bevorzugt verwendet wird.

Beispiele geeigneter erfindungsgemäß zu verwendender, gegebenenfalls substituierter $C_6$-$C_{10}$-Arylreste sind Phenyl- oder Naphthylreste, von denen die Phenylreste vorteilhaft sind und deshalb bevorzugt verwendet werden.

Beispiele geeigneter erfindungsgemäß zu verwendender, gegebenenfalls substituierter $C_7$-$C_{20}$-Arylalkylreste sind Phenylmethyl-, 2- Phenyl-ethyl- oder 3- Phenylpropylreste, von denen die 2-Phenyl-ethylreste vorteilhaft sind und deshalb bevorzugt verwendet werden.

Beispiele geeigneter erfindungsgemäß zu verwendender, gegebenenfalls substituierter $C_7$-$C_{20}$-Alkylarylreste sind 2-, 3- und 4-Methyl-phen-1-yl-, 4-Butyl-phen-1-yl-, 4-Butyl-2-methyl-phen-1-yl-, 2,4- Dimethyl-phen-1-yl- oder 4-Octyl-phen-1-yl-Reste, von denen die 4-Butyl-phen-1-yl- oder 4-Octyl-phen-1-yl- Reste vorteilhaft sind und deshalb bevorzugt verwendet werden.

Beispiele geeigneter erfindungsgemäß zu verwendender $C_{11}$-$C_{26}$-Arylcycloalkylreste sind 2- oder 3-Phenyl-cyclopent-1-yl- oder 2-, 3- oder 4-Phenyl-cyclohex-1-yl-Reste, von denen der 4-Phenyl-cyclohex-1-yl-Rest vorteilhaft ist und deshalb bevorzugt verwendet wird.

Beispiele geeigneter erfindungsgemäß zu verwendender $C_{11}$-$C_{26}$-Cycloalkylarylreste sind 2-, 3- oder 4-Cyclopentyl-phen-1-yl- oder 2-, 3- oder 4-Cyclohexyl-phen-1-yl-Reste, von denen der 4-Cyclohexyl-phen-1-yl-Rest vorteilhaft ist und deshalb bevorzugt verwendet wird.

Die Reste $R^3$ bezeichnen $C_1$-$C_4$-Alkylreste oder Nitrilgruppen -CN. Beispiele geeigneter erfindungsgemäß zu verwendender $C_1$-$C_4$-Alkylreste sind Methyl-, Ethyl-, Propyl- oder Butylreste, von denen der Methylrest vorteilhaft ist und deshalb bevorzugt verwendet wird.

Die Reste $R^4$ bezeichnen gegebenenfalls substituierte $C_1$-$C_4$-Alkylreste; Beispiele geeigneter erfindungsgemäß zu verwendender Alkylreste dieser Art sind die vorstehend genannten. Beispiele geeigneter erfindungsgemäß zu verwendender Substituenten sind die vorstehend bei den Resten $R^2$ aufgeführten.

Bei dem Rest $R^1$ der linearen oder verzweigten Gruppe $-R^1-(-Z^1)_u$, worin der Index u = 1 oder 2, handelt es sich um einen zwei- oder dreibindigen Rest, welcher sich von den folgenden Verbindungen ableitet:

(i) einem Alkan, Alken, Cycloalkan, Cycloalken, Alkylcycloalkan, Alkylcycloalken, Alkenylcycloalkan, oder Alkenylcycloalken, Aromaten und Heteroamaten sowie einem Alkyl-, Alkenyl-, Cycloalkyl-, Cycloalkenyl-, Alkylcyloalkyl-, Alkylcycloalkenyl-, Alkenylcycloalkyl- oder Alkenylcycloalkenyl-substitiuierten Aromaten oder Heteroaromaten; oder von

(ii) einem vorstehend genannten Rest, welcher mindestens ein Heteroatom in der Kette und/oder im Rest enthält; oder von

(iii) einem unter (i) oder (ii) genannten Rest, dessen Kette und/oder Ring substituiert ist.

Beispiele geeigneter erfindungsgemäß zu verwendender Reste R1 sind die nachstehend aufgeführten Reste (i1) bis (iii2), von denen die Reste (i2) bis (i5) sowie (i20) und (i21) vorteilhaft sind und deshalb besonders bevorzugt verwendet werden.

$-(CH_2)_1-$ (i1)  $-(CH_2)_2-$ (i2)  $-(CH_2)_3-$ (i3)  $-(CH_2)_4-$ (i4)  $-(CH_2)_5-$ (i5)

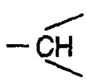
(i6)

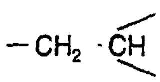
(i7)

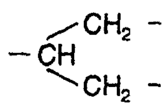
(i8)

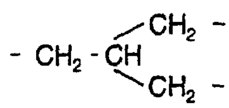
(i9)

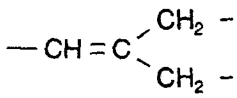 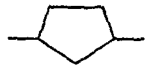 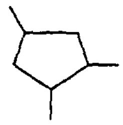 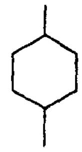

(i10) (i11) (i12) (i13)

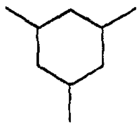 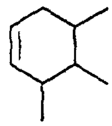 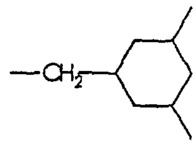 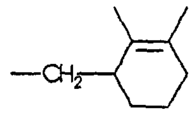

(i14) (i15) (i16) (i17)

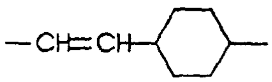 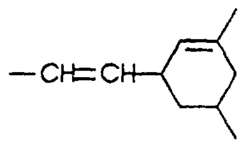 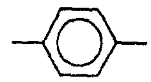

(i18) (i19) (i20)

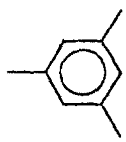 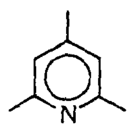 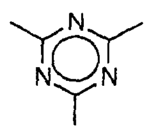 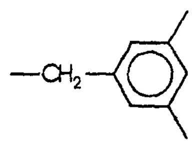

(i21) (i22) (i23) (i24)

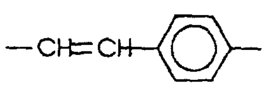 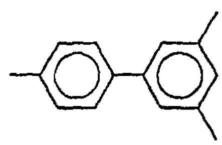

(i25) (i26)

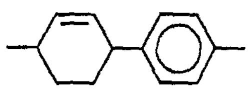 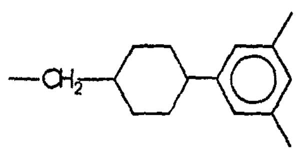

(i27) (i28)

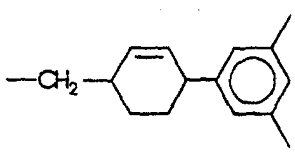 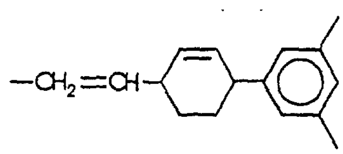

(i29) (i30)

$-CH_2-CH_2-O-CH_2-CH_2-$

(ii1)

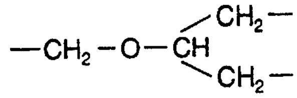

(ii2)

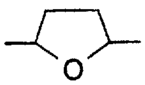

(ii3)

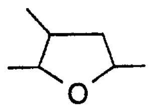

(ii4)

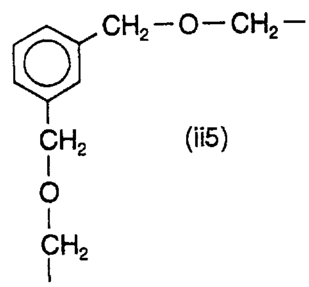

(ii5)

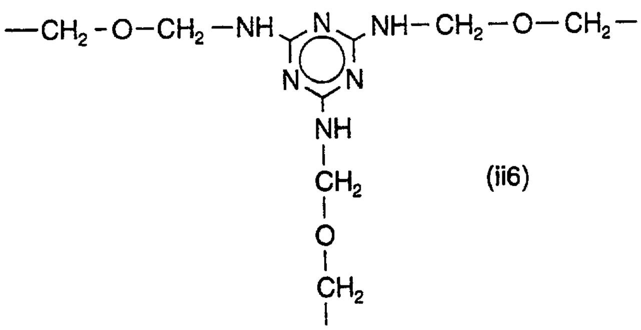

(ii6)

(iii1)

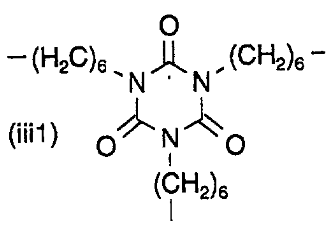

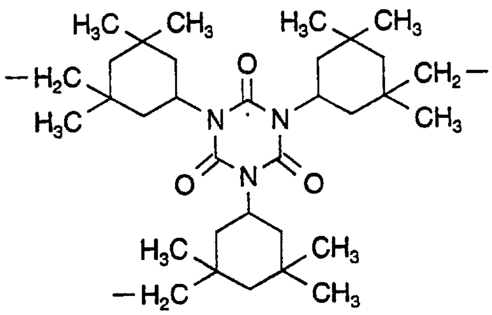

(iii2)

Beispiele geeigneter erfindungsgemäß zu verwendender Substituenten für die Reste $R^1$ sind die vorstehend bei den Resten $R^2$ aufgeführten.

Als Variable $Z^1$ der linearen oder verzweigten Gruppe $-R^1-(-Z^1)_u$, worin der Index u = 1 oder 2, kommen alle reaktiven funktionellen Gruppen der organischen Chemie in Betracht, welche unter den üblichen und bekannten Bedingungen Reaktionen eingehen, welche zu dem gewünschten weiteren Aufbau der erfindungsgemäßen Verbindung oder zu ihrer Verknüpfung mit anderen Verbindungen, wie etwa mit den von den Beschichtungsmitteln oder den Klebstoffen her bekannten Vernetzern. Beispiele für erfindungsgemäß besonders vorteilhafte Gruppen $-Z^1$ sind:

$-OH$, $-NH_2$, $-SH$,
$-COOH$, $-SO_3H$, $-PO_3H$
$-O-C(O)-NH_2$,
$-O-C(O)-NH-C(O)-NH_2$,
$-NCO$;
$-NH-C(O)-OR^2$,
$-NH-C(O)-NH-C(O)-OR^2$,
$-O-C(O)-CH_2-C(O)-R^2$
$-O-C(O)-CH_2-C(O)-OR^2$
$-O-C(O)-CR^3 = CH_2$,

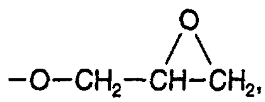

$-O-Si-(OR^4)_3$,
$-O-(-CH_2-)_v-CH = CH_2$ und
$-O-C(O)-(-CH_2-)_v-CH = CH2$; worin der Index v fiir 0 steht oder für eine ganze Zahl von 1 bis 3, insbesondere aber für 0 und 1;
$-O-C(O)-CH=CH-C(O)-O-R^2$,
$-O-C(O)-CH=CH-CH=CH-R^2$ und
$-O-C(O)-CH=CH-CH_2-CH=CH-R^2$.

Die vorstehend im Detail beschriebenen reaktiven funktionellen Gruppen der allgemeinen Formel II sind erfindungsgemäß über Abstandshaltergruppen mit der Zentralgruppe I verbunden.

Erfindungsgemäß sind als Abstandshaltergruppen alle zweibindigen organischen Reste geeignet.

Beispiele gut geeigneter zweibindiger organischer Reste sind die vorstehend beschriebenen zweibindigen Reste $R^1$, insbesondere aber der Rest (i5).

Weitere Beispiele gut geeigneter erfindungsgemäß zu verwendender zweibindiger organischer Reste sind die nachstehend aufgeführten Abstandshaltergruppen (iv1) bis (iv8). Diese weisen hinsichtlich ihrer Herstellbarkeit und den vorteilhaften Eigenschaften, welche sie den erfindungsgemäßen Verbindungen mitteilen, besondere Vorteile auf, weswegen sie bevorzugt verwendet werden. Von den Abstandshaltergruppen (iv1) bis (iv8) sind die Abstandshaltergruppen (iv1) und (iv7) ganz besonders vorteilhaft und werden deshalb ganz besonders bevorzugt verwendet.

(IVA) 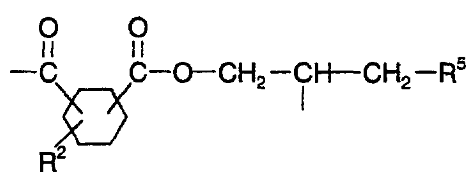

(IVB) 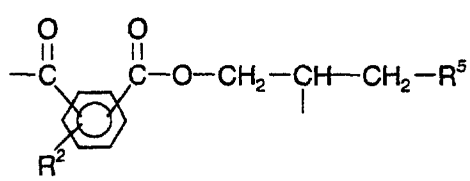

(iv7) 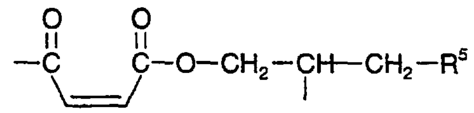

(iv8) 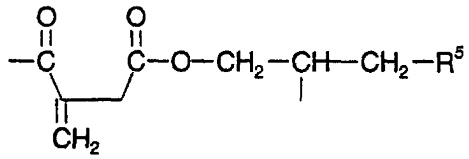

(iv9) 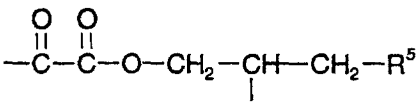

oder

(iv10) 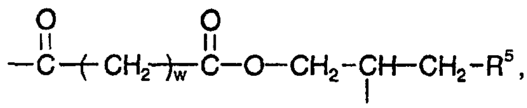

worin der Index w = eine ganze Zahl von 1 bis 10.

In den allgemeinen Formeln IVA und IVB hat der Rest $R^2$ die vorstehend angegebene Bedeutung. Die Carbonyloxy-Substituenten der Ringe in der allgemeinen Formeln IVA stehen in 1,2- (Abstandshaltergruppe iv1), 1,3- (Abstandshaltergruppe iv2) oder 1,4-Stellung (Abstandshaltergruppe iv3) zueinander. Die Carbonyloxy-Substituenten der Ringe in der allgemeinen Formeln IVb stehen ebenfalls in 1,2-(Abstandshaltergruppe iv4), 1,3- (Abstandshaltergruppe iv5) oder 1,4-Stellung (Abstanshaltergruppe iv6) zueinander.

In den Formeln der Abstandshaltergruppen (iv1) bis (iv10) bezeichnet der Rest $R^5$ Alkyl-, Cycloalkyl- oder Arylether- oder Alkyl-, Cycloalkyl- oder Arylcarbonyloxi-Reste, insbesondere aber tertiäre Alkylcarbonyloxireste. Beispiel geeigneter erfindungsgemäß zu verwendender Reste $R^5$ sind insbesondere Versatic$^R$-Säurereste, d. h. Reste tertiärer, stark verzweigter Monocarbonsäuren.

Erfindungsgemäß ist die vorstehend im Detail beschriebene reaktive funktionelle Gruppe II mit den Abstandshaltergruppen (iv1) bis (iv10) über das sekundäre Kohlenstoffatom verknüpft.

Die ganz besonders vorteilhaften erfindungsgemäßen Verbindungen weisen den besonderen Vorteile auf, daß ihre Hydroxylgruppen in außerordentlich vielfältiger Weise in andere reaktive funktionelle Gruppen II umgewandelt werden können. Beispiele für solche reaktive funktionelle Gruppen II sind die vorstehend beschriebenen Gruppen -Z. Sie können beispielsweise durch die Umsetzung der Hydroxylgruppen mit den folgenden Verbindungen hergestellt werden:

$R^2O\text{-}C(O)\text{-}NH_2$,
$R^2O\text{-}C(O)\text{-}NH\text{-}C(O)\text{-}NH_2$,
$Cl\text{-}C(O)\text{-}OR^2$,
$Cl\text{-}C(O)\text{-}NH\text{-}C(O)\text{-}OR^2$,
$Cl\text{-}C(O)\text{-}CH_2\text{-}C(O)\text{-}R^2$
$Cl\text{-}C(O)\text{-}CH_2\text{-}C(O)\text{-}OR^2$
$HO\text{-}C(O)\text{-}CR^3 = CH_2$,

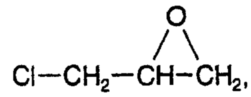

Cl-Si-$(OR^4)_3$,
Cl-$(-CH_2-)_v$-CH = $CH_2$ und
Cl-C(O)-$(-CH_2-)_v$-CH = $CH_2$; worin der Index v gleich 0 ist oder für eine ganze Zahl von 1 bis 3, insbesondere aber für 0 and 1;
Cl-C(O)-CH=CH-C(O)-O-$R^2$,
Cl-C(O)-CH=CH-CH=CH-$R^2$ und
Cl-C(O)-CH=CH-$CH_2$-CH=CH-$R^2$.

Hierin haben die Indices und die Variablen die vorstehend angegebene Bedeutung.

Überdies haben die ganz besonders vorteilhaften erfindungsgemäßen Verbindungen, welche die Abstandshaltergruppen (iv7) und (iv8) aufweisen, den ganz besonderen Vorteil, daß sie an ihrer olefinischen Doppelbindung modifiziert werden können, so daß sie außer den reaktiven funktionellen Gruppen II noch weitere reaktive funktionelle Gruppen enthalten. Ein Beispiel für eine solche Modifizierung ist die Addition von Aminen an die Doppelbindungen.

Beispiele für ganz besonders vorteilhafte erfindungsgemäße Verbindung sind demnach die Verbindungen (I1) bis (I3).

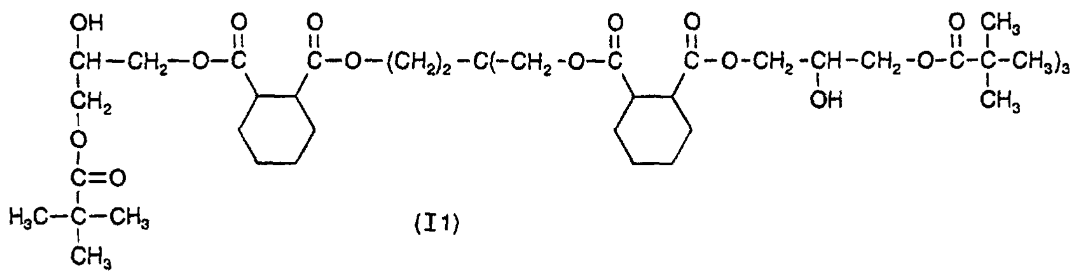

(I1)

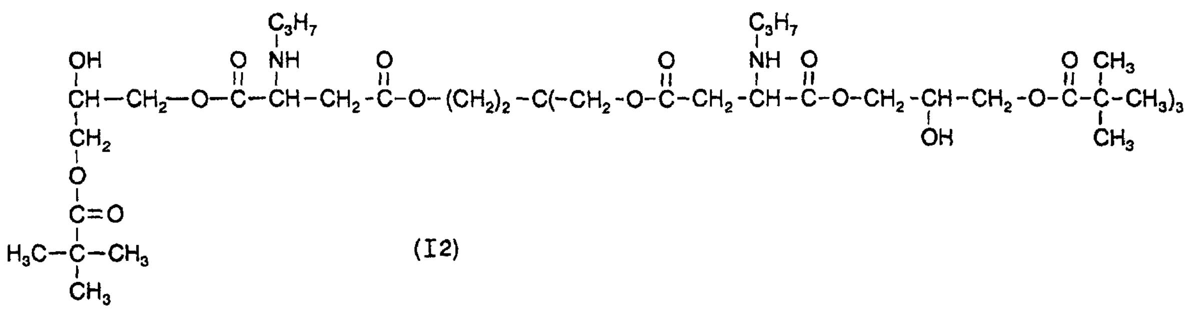

(I2)

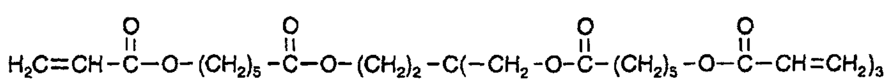

(I3)

Die Herstellung der erfindungsgemäßen Verbindungen kann nach den üblichen und bekannten Methoden der Herstellung hyperverzweigter und dendrimer Verbindungen erfolgen. Geeignete Synthesemethoden werden beispielsweise in den Patentschriften WO 93/17060 oder WO 96/12754 oder in dem Buch von G. R. Newkome, C. N. Moorefield und F. Vögtle, "Dendritic Molecules, Concepts, Syntheses, Perspectives", VCH, Weinheim, New York, 1996, beschrieben. Geeignete Ausgangsverbindungen kann der Fachmann daher leicht anhand der gewünschten Strukturen

der erfindungsgemäßen Verbindungen auswählen.

Erfindungswesentlich ist es, die Zentralgruppe I bzw. die erfindungsgemäße Verbindung mit Hilfe einer tetrafunktionellen Verbindung der allgemeinen Formen III

$$\mathbf{C[-A_q\text{-}XH]_m[-A_r\text{-}XH]_n[-A_s\text{-}XH]_o[-A_t\text{-}XH]_p} \quad \text{(III),}$$

herzustellen. Im folgenden wird diese Verbindung der Kürze halber als "Verbindung III" bezeichnet.

In der allgemeinen Formel III haben die Indices und die Variablen dieselbe Bedeutung wie vorstehend bei der allgemeinen Formel I angegeben. Erfindungsgemäß ist es von besonderem Vorteil, wenn die Variable X ein Sauerstoffatom -O- darstellt.

Demgemäß sind für die Herstellung der Zentralgruppe I bzw. der erfindungsgemäßen Verbindungen die Tetrole der allgemeinen Formel III von besonderem Vorteil und werden deshalb besonders bevorzugt verwendet. Im folgenden werden sie der Kürze halber als "Tetrole III" bezeichnet.

Beispiele ganz besonders gut geeigneter erfindungsgemäß zu verwendender Tretrole III sind die Tretrole (III1) bis (III10):

$$HO\text{-}(\text{-}CH_2\text{-})_2\text{-}C(\text{-}CH_2\text{-}OH)_3, \quad \text{(III1)}$$

$$HO\text{-}(\text{-}CH_2\text{-})_3\text{-}C(\text{-}CH_2\text{-}OH)_3, \quad \text{(III2)}$$

$$HO\text{-}(\text{-}CH_2\text{-})_4\text{-}C(\text{-}CH_2\text{-}OH)_3, \quad \text{(III3)}$$

$$HO\text{-}(\text{-}CH_2\text{-})_5\text{-}C(\text{-}CH_2\text{-}OH)_3, \quad \text{(III4)}$$

$$[HO\text{-}(\text{-}CH_2\text{-})_2\text{-}]_2C(CH_2\text{-}OH)_2, \quad \text{(III5)}$$

$$[HO\text{-}(\text{-}CH_2\text{-})_2\text{-}]_3C\text{-}CH_2\text{-}OH, \quad \text{(III6)}$$

$$HO\text{-}(\text{-}CH_2\text{-})_3\text{-}C[\text{-}(\text{-}CH_2\text{-})_2\text{-}OH]_3, \quad \text{(III7)}$$

$$HO\text{-}(\text{-}CH_2\text{-})_3\text{-}C[\text{-}(\text{-}CH_2\text{-})_2\text{-}OH]_2(\text{-}CH_2\text{-}OH), \quad \text{(III8)}$$

$$HO\text{-}(\text{-}CH_2\text{-})_4\text{-}C(\text{-}CH_2\text{-}OH)\ [\text{-}(\text{-}CH_2\text{-})_2\text{-}OH]\ [\text{-}(\text{-}CH_2\text{-})_3\text{-}OH] \text{ oder} \quad \text{(III9)}$$

$$HO\text{-}(\text{- }CH_2\text{-})_5\text{-}C(\text{-}CH_2\text{-}OH)[\text{-}(\text{-}CH_2\text{-})_4\text{-}OH]2 \quad \text{(III10).}$$

Von diesen ist das Tetrol (III1) (2,2-Bis-hydroxymethyl-butandiol-(1,4); Homopentaerythrit) besonders hervorzuheben, weil es den erfindungsgemäßen Verbindungen ganz besonders vorteilhafte Eigenschaften vermittelt. Es wird deshalb ganz besonders bevorzugt verwendet.

In erfindungsgemäßer Verfahrensweise resultieren gemäß einer ersten Variante die reaktiven funktionellen Gruppen II und damit die erfindungsgemäßen Verbindungen aus der Umsetzung der Verbindungen III, insbesondere der Tetrole III, mit geeigneten organischen Verbindungen in einer Stufe.

Als organische Verbindungen kommen alle Verbindungen in Betracht, welche zum einen funktionelle Gruppen tragen, die mit dem vorstehenden Detail beschriebenen Gruppen -XH reagieren können, und zum anderen die vorstehend im Detail beschriebenen zweibindigen Reste $R^1$ in die Zwischenstufe einführen.

Beispiele geeigneter erfindungsgemäß zu verwendender organischer Verbindungen sind demnach die vorstehenden Detail beschriebenen zweibindigen Reste $R^1$, welche insbesondere die folgenden funktionellen Gruppen tragen:

-NCO,
-Cl,
-Br,
-C(O)-Cl,
-C(O)-Br,
-C(O)-OH oder
-C(O)-O-C(O)-.

Von diesen sind die Carboxylgruppen und die Anhydridgruppen von besonderem Vorteil und werden deshalb besonders bevorzugt verwendet.

Der Fachmann kann daher die geeigneten erfindungsgemäß zu verwendenden organischen Verbindungen anhand der gewünschten Zielstruktur der erfindungsgemäßen Verbindung in einfacher Weise auswählen.

In erfindungsgemäßer Verfahrensweise kann in einer zweiten Variante aus den Verbindungen III, insbesondere den Tetrolen III, und einer geeigneten organischen Verbindung zuerst eine Zwischenstufe hergestellt werden, welche die Zentralgruppe I und vier Abstandshaltergruppen aufweist. Die geeignete organische Verbindung wird so gewählt, daß die resultierenden Abstandshaltergruppen jeweils eine reaktive funktionelle Gruppe tragen. Diese reaktiven funktionellen Gruppen werden in einer zweiten Stufe mit einer geeigneten organischen Verbindung unter Bildung der reaktiven funktionellen Gruppen II und damit der erfindungsgemäßen Verbindungen umgesetzt. Erfindungsgemäß wird dieser Variante der Vorzug gegeben.

Beispiele geeigneter organischer Verbindungen sind die vorstehend bei der ersten Variante auf geführten Verbindungen.

Beispiele geeigneter reaktiver funktioneller Gruppen sind die vorstehend bei der ersten Variante aufgeführten funktionellen Gruppen.

Beispiele von organischen Verbindungen, welche sich für diese Variante gut eignen, sind epsilon-Caprolacton, Hexahydrophthalsäure, Hexahydrophthalsäureanhydrid, Phthalsäure, Phthalsäureanhydrid, Hexahydroterephthalsäure, Terephthalsäure, Fumarsäure, Maleinsäure, Maleinsäureanhydrid, Itaconsäure, Itaconsäureanhydrid, Oxalsäure, Malonsäure, Malonsäureanhydrid, Bernsteinsäure, Bernsteinsäureanhydrid, Glutarsäure, Glutarsäureanhydrid, Adipinsäure, Adipinsäureanhydrid, Pimelinsäure, Korksäure, Azelainsäure, Sebacinsäure oder Decan-, Undecan- oder Dodecandicarbonsäure. Von diesen sind epsilon-Caprolacton, Maleinsäure oder Maleinsäureanhydrid und Hexahydrophthalsäureanhydrid besonders gut geeignet und werden deswegen erfindungsgemäß besonders bevorzugt verwendet.

Beispiele gut geeigneter organischer Verbindungen, welche in der zweiten Stufe mit der Zwischenstufe unter Bildung der reaktiven funktionellen Gruppen II und damit der erfindungsgemäßen Verbindungen umgesetzt werden können, sind epoxidgruppenhaltige, insbesondere glycidylgruppenhaltige, Verbindungen.

Beispiele und geeigneter epoxidgruppenhaltiger, insbesondere glycidylgruppenhaltiger, Verbindungen sind Ethylenoxid, Propylenoxid, Epichlorhydrin, Glycidol, Glycidylether, insbesondere Aryl- und Alkylglycidylether, oder Glycidylester, insbesondere die Glycidylester von tertiären, stark verzweigten, gesättigten Monocarbonsäuren, welche unter dem Handelsnamen Versatic$^R$-Säuren von der Firma Deutsche Shell Chemie vertrieben werden. Von diesen sind die Versatic$^R$-Säureglycidylester, welche unter dem Handelsnamen Cardura$^R$ E10 verrtieben werden, ganz besonders vorteilhaft und werden deshalb ganz besonders bevorzugt verwendet.

Weitere Beispiele gut geeigneter organischer Verbindungen, welche in der zweiten Stufe mit der Zwischenstufe unter Bildung der reaktiven funktionellen Gruppen II und damit der erfindungsgemäßen Verbindungen umgesetzt werden können, sind Acrylsäure und Methacrylsäure.

Alle Varianten des erfindungsgemäßen Verfahrens können in Lösung oder in Masse durchgeführt werden. Je nach vorliegenden Reaktionspartnern kann unter Druck gearbeitet werden. Im allgemeinen liegen die geeigneten Reaktionstemperaturen bei -50 bis + 300 °C, vorzugsweise 0 bis 250 °C, vorzugsweise Raumtemperatur bis 200 °C und insbesondere 60 bis 160 °C. Die jeweils besonders gut geeigneten Reaktionsbedingungen kann der Fachmann leicht anhand der Eigenschaften der Reaktionspartner auswählen.

Die erfindungsgemäßen Verbindungen, insbesondere die in erfindungsgemäßer Verfahrensweise hergestellten, weisen besondere Vorteile auf. So sind in zahlreichen Anwendungszwecken den bekannten hyperverzweigten Verbindungen und Dendrimeren überlegen, so daß sie sehr viel breiter als diese verwendbar sind.

So gestatten sie in einfacher Weise die Herstellung neuer Dendrimere höherer Generationen.

Insbesondere ermöglichen die erfindungsgemäßen Verbindungen ganz allgemeinen die Herstellung von flüssigen, festen oder von in wäßrigen oder organischen Medien gelösten oder dispergierten erfindungsgemäßen Mehrstoffmischungen, welche neue vorteilhafte anwendungstechnische Eigenschaftsprofile aufweisen. So werden mit Hilfe

der erfindungsgemäßen Verbindungen in wäßrigen oder organischen Medien gelöste oder dispergierte Beschichtungsmittel, Klebstoffe oder Dichtungsmassen erhalten, welche besonders hohe Festkörpergehalte aufweisen. Außerdem weisen erfindungsgemäße Pulverlacke und Pulverslurries vorteilhaft niedrige Filmbildungstemperaturen auf, welche nicht zu nahe an der Vernetzungstemperatur liegen. Überdies hinaus sind die erfindungsgemäßen Beschichtungsmittel für die Herstellung von Folien, insbesondere von freitragenden Lackfilmen geeignet.

Die erfindungsgemäßen Beschichtungsmittel, Klebstoffe und Dichtungsmassen können luftrocknend, physikalisch trocknend, thermisch härtbar, mit aktinischem Licht härtbar und/oder mit Elektronenstrahlung härtbar sein. Hierbei weisen die thermisch härtbaren erfindungsgemäßen Beschichtungsmitteln, Klebstoffe und Dichtungsmassen besondere Vorteile auf.

Bei den erfindungsgemäßen Beschichtungsmitteln kann es sich um in wäßrigen oder organischen Medien gelöste oder dispergierte Spritzlacke, flüssige Lacke, Pulverlacke oder Pulverslurries handeln. Diese eignen sich hervorragend für alle Anwendungszwecke, denen Lacke üblicherweise zugeführt werden. Insbesondere eignen sie sich als Lacke für den Industriesektor, Möbellacke, Bautenanstrichmittel, Automobilserienlacke oder als Autoreparaturlacke.

Ganz besondere Vorteile weisen die erfindungsgemäßen Beschichtungsmitteln auf, welche erfindungsgemäße Verbindungen enthalten, die als reaktive funktionelle Gruppen II Hydroxylgruppen enthalten.

Neben den erfindungsgemäßen Verbindungen enthalten die erfindungsgemäßen Beschichtungsmittel der genannten Art Bindemittel, welche ebenfalls Hydroxylgruppen enthalten.

Als hydroxyfunktionelle Bindemittel kommen vorzugsweise Bindemittel auf Basis von Polyacrylaten, Polyestern, Polyurethanen, acrylierten Polyurethanen, acrylierten Polyestern, Polylactonen, Polycarbonaten, Polyethern und/oder (Meth)Acrylatdiolen in Betracht. Hydroxyfunktionelle Bindemittel sind dem Fachmann bekannt, und zahlreiche geeignete Beispiele sind marktgängig.

Vorzugsweise werden Polyacrylate, Polyester und/oder Polyurethane verwendet. Beispiele für besonders bevorzugt verwendete Bindemittel dieser Art sind:

1. Polyacrylate mit einer Hydroxylzahl von 40 bis 240, vorzugsweise 60 bis 210, insbesondere 100 bis 200, einer Säurezahl von 0 bis 35,
Glasübergangstemperaturen von -35 bis + 85 °C und zahlenmittlere Molekulargewichte $M_n$ von 1500 bis 300.000.

Die Glasübergangstemperaturen der Polyacrylate wird bekanntermaßen durch Art und Menge der eingesetzten Monomere bestimmt. Die Auswahl der Monomeren kann von Fachmann unter Zuhilfenahme der folgenden Formel V getroffen werden, nach welcher die Glasübergangstemperaturen näherungsweise berechnet werden können.

$$1 / Tg = \sum_{n=1}^{n=x} W_n / Tg_n ; \quad \sum_n W_n = 1 \qquad (V)$$

Tg = Glasübergangstemperatur des Polyacrylatharzes
$W_n$ = Gewichtsanteil des n-ten Monomers
$Tg_n$ = Glasübergangstemperatur des Homopolymers aus dem n-ten Monomer
x = Anzahl der verschiedenen Monomeren

Maßnahmen zur Steuerung des Molekulargewichts (z. B. Auswahl entsprechender Polymerisationinitiatoren, Verwendung von Kettenübertragungsmitteln oder spezieller Verfahren der Polymerisation usw.) gehören zum Fachwissen und müssen hier nicht näher erläutert werden.

1.1 Besonders bevorzugte Polyacrylate sind herstellbar indem (a1) 10 bis 92, vorzugsweise 20 bis 60 Gew.-%, eines Alkyl- oder Cycloalkylmethacrylats mit 1 bis 18, vorzugsweise 4 bis 13 Kohlenstoffatomen im Alkyl- oder Cycloalkylrest oder Mischungen aus solchen Monomeren, (a2) 8 bis 60, vorzugsweise 12,5 bis 50,0 Gew.-%, eines Hydroxyalkylacrylats oder eines Hydroxyalkylmethacrylats mit 2 bis 4 Kohlenstoffatomen im Hydroxyalkylrest oder Mischungen aus solchen Monomeren, (a3) 0 bis 5, vorzugsweise 0,7 bis 3 Gew.-% Acrylsäure oder Methacrylsäure oder Mischungen aus diesen Monomeren und (a4) 0 bis 50, vorzugsweise bis zu 30 Gew.-%, von von (a1), (a2) und (a3) verschiedenen, mit (a1), (a2) und (a3) copolymerisierbaren ethylenisch ungesättigten Monomeren oder Mischungen aus solchen Monomeren zu Polyacrylaten der vorstehend angegebenen Spezifikation polymerisiert werden.

Beispiele geeigneter (a1)-Komponenten sind Methyl-, Ethyl-, Propyl-, n-Butyl-, Isobutyl-, tert.-Butyl-, Pentyl-, Hexyl-, Heptyl- oder 2-Ethylhexylacrylat oder -methacrylat sowie Cyclohexyl-, tert.-Butylcyclohexyl- oder

Isobornylacrylat oder -methacrylat.

Beispiele geeigneter (a2)-Komponenten sind Hydroxyethyl-, Hydroxypropyloder Hydroxybutyl- oder Hydroxymethylcyclohexylacrylat oder -methacrylat oder Addukte von (Meth)Acrylsäure und Epoxiden wie Versaticsäure[R]glycidylester.

Beispiele geeigneter (a4)-Komponenten sind Vinylaromaten wie Styrol, Vinyltoluol, alpha- Methylstyrol, alpha-Ethylstyrol, kernsubstituierte Diethylstyrole, Isopropylstyrol, Butylstyrol und Methoxystyrole; Vinylether wie Ethylvinylether, n-Propylvinylether, Isopropylvinylether, n-Butylvinylether oder Isobutylvinylether; Vinylester wie Vinylacetat, Vinylpropionat, Vinylbutyrat, Vinylpivalat oder der Vinylester der 2-Methyl-2-ethylheptansäure; oder Allylether wie Trimethylolpropanmono-, -di- oder - triallylether oder ethoxilierter oder propoxilierter Allylalkohol.

1.2 Weitere Beispiele besonders bevorzugter Polyacrylate werden in der europäischen Patentanmeldung EP-A-0 767 185 und den amerikanischen Patentschriften US-A- 5 480 493, 5 475 073 oder 5 534 598 beschrieben.

1.3 Weitere Beispiele besonders bevorzugter Polyacrylate werden unter der Marke Joncryl[R] vertrieben, wie etwa Joncryl[R] SCX 912 und 922,5.

1.4 Weitere Beispiele für besonders bevorzugte Polyacrylate sind solche, welche erhältlich sind, indem (a1) 10 bis 51 Gew.-%, vorzugsweise 25 bis 41 Gew.-%, 4-Hydroxy-n-butylacrylat oder -methacrylat oder eine Mischung hiervon, insbesondere aber 4-Hydroxy-n-butylacrylat, (a2) 0 bis 36 Gew.-%, vorzugsweise 0,1 bis 20 Gew.-%, eines von (a1) verschiedenen hydroxylgruppenhaltigen Esters der Acrylsäure oder der Methacrylsäure oder eines Gemisches hiervon, (a3) 28 bis 85 Gew.-%, vorzugsweise 40 bis 70 Gew.-%, eines von (a1) und (a2) verschiedenen, aliphatischen oder cycloaliphatischen Esters der Methacrylsäure mit mindestens vier Kohlenstoffatomen im Alkoholrest oder eines Gemisches solchen Monomeren, (a4) 0 bis 3 Gew.-%, vorzugsweise 0,1 bis 2 Gew.-%, einer ethylenisch ungesättigten Carbonsäure oder einer Mischung aus solchen Säuren und (a5) 0 bis 20 Gew.-%, vorzugsweise 5 bis 15 Gew.-%, eines von (a1), (a3) und (a4) verschiedenen ungesättigten Monomeren oder einer Mischung aus solchen Monomeren zu einem Polyacrylat mit einer Hydroxylzahl von 60 bis 200, vorzugsweise von 100 bis 160, einer Säurezahl von 0 bis 35 und einem zahlenmittleren Molekulargewicht $M_n$ von 1500 bis 10000 polymerisiert werden, wobei die Zusammensetzung der Komponente (a3) so gewählt wird, daß bei alleiniger Polymerisation dieser Komponente (a3) ein Polymethacrylat einer Glasübergangstemperatur von + 10 bis + 100 °C, vorzugsweise von + 20 bis + 60 °C, erhalten wird.

Beispiele geeigneter Komponenten (a2) sind Hydroxialkylester der Acrylsäure und Methacrylsäure wie Hydroxyethyl- oder Hydroxypropylacrylat oder - methacrylat, wobei die Wahl so zu treffen ist, daß bei alleiniger Polymerisation dieser Komponente (a2) ein Polyacrylat einer Glasübergangstemperatur von 0 bis + 80 °C, vorzugsweise von + 20 bis + 60 °C erhalten wird.

Beispiele geeigneter Komponenten (a3) sind aliphatische Ester der Methacrylsäure mit vier bis 20 Kohlenstoffatomen in Alkoholrest wie n-Butyl -, Isobutyl -, tert. - Butyl -, 2-Ethylhexyl-, Stearyl- und Laurylmethacrylat; oder cycloaliphatische Ester der Methacrylsäure wegen Cyclohexylmethacrylat.

Beispiele geeigneter Komponenten (a4) sind Acrylsäure und/oder Methacrylsäure.

Beispiele geeigneter Komponenten (a5) sind vinylaromatische Kohlenwasserstoffe wie Styrol, alpha-Alkylstyrol oder Vinyltoluol; Amide der Acrylsäure und Methacrylsäure wie Methacrylamid und Acrylamid; Nitrile der Acrylsäure und Methacrylsäure; Vinylether oder Vinylester, wobei die Zusammensetzung dieser Komponente (a5) vorzugsweise so zutreffend ist, daß bei alleiniger Polymerisation der Komponenten (a5) ein Polyacrylat mit einer Glasübergangstemperatur von + 70 bis + 120 °C, insbesondere von + 80 bis + 100 °C resultiert.

1.5 Die Herstellung dieser Polyacrylate ist allgemein bekannt und wird beispielsweise in dem Standardwerk Houben-Weyl, Methoden der organischen Chemie, 4. Auflage, Band 14/1, Seiten 24 bis 255, 1961, beschrieben.

2. Polyesterharze, welche herstellbar sind, indem (a1) mindestens eine cycloaliphatische oder aliphatische Polycarbonsäure, (a2) mindestens ein aliphatisches oder cycloaliphatisches Polyol mit mehr als zwei Hydroxylgruppen im Molekül, (a3) mindestens ein aliphatisches oder cycloaliphatisches Diol und (a4) mindestens eine aliphatische, lineare oder verzweigte gesättigte Monocarbonsäure in einem molaren Verhältnis von (a1) : (a2) : (a3) : (a4) = 1,0 : 0,2 bis 1,3 : 0,0 bis 1,1 : 0,0 bis 1,4, vorzugsweise 1,0 : 0,5 bis 1,2 : 0,0 bis 0,6 : 0, 2 bis 0,9 zu einem Polyester oder Alkydharz umgesetzt werden.

Beispiele geeigneter Komponenten (a1) sind Hexahydrophthalsäure, 1,4-Cyclohexandicarbonsäure, Endomethylentetrahydrophthalsäure, Oxalsäure, Malonsäure, Bernsteinsäure, Glutarsäure, Adipinsäure, Pimelinsäure, Korksäure, Azelainsäure oder Sebacinsäure.

Beispiele geeigneter Komponenten (a2) sind Pentaerythrit, Trimethylolpropan, Triethylolethan und Glycerin.

Beispiele geeigneter Komponenten (a3) sind Ethylenglykol, Diethylenglykol, Propylenglykol, Neopentylglykol, 2-Methyl-2-propylpropandiol-1,3, 2-Methyl-2-butylpropandiol-1,3, 2,2,4-Trimethylpentandiol-1,5, 2,2,5-Trimethylhexandiol-1,6, Hydroxypivalinsäureneopentylglykolester oder Dimethylolcyclohexan.

Beispiele geeigneter Komponenten (a4) sind 2-Ethylenhexansäure, Laurinsäure, Isooctansäure, Isononansäure oder Monocarbonsäuremischungen, welche aus Kokosfett oder Palmkernfett gewonnen werden.

Die Herstellung der erfindungsgemäß bevorzugt verwendeten Polyester und Alkydharze ist allgemein bekannt und wird beispielsweise in dem Standardwerk Ullmanns Encyklopädie der technische Chemie, 3. Auflage, Band 14, Urban & Schwarzenberg, München, Berlin, 1963, Seiten 80 bis 89 und Seiten 99 bis 105, sowie in den Büchern: "Résines Alkydes-Polyesters" von J. Bourry, Paris, Verlag Dunod, 1952, "Alkyd Resins" von C. R. Martens, Reinhold Publishing Corporation, New York, 1961, sowie "Alkyd Resin Technology" von T. C. Patton, Intersience Publishers, 1962, bschrieben.

3. Polyurethane, wie sie in den Patentschriften EP-A-0 708 788, DE-A-44 01 544 oder DE-A-195 34 361 beschrieben werden.

Zur weiteren Erhöhung des Festkörpergehalts kann ein Teil der Bindemittel durch Reaktivverdünner ersetzt sein.

Beispiele geeigneter Reaktiverdünner sind verzweigte, cyclische und/oder acyclische $C_9$-$C_{16}$-Alkane, die mit mindestens zwei Hydroxyl- oder Thiolgruppen oder mindestens einer Hydroxyl- und mindestens einer Thiolgruppe funktionalisiert sind, insbesondere Diethyloctandiole.

Weitere Beispiele für geeignete Reaktivverdünner sind oligomere Polyole, welche aus Oligomeren, die durch Metathesereaktionen von acyclischen Monoolefinen und cyclischen Monoolefinen gewonnen werden, durch Hydroformylierung und anschließender Hydrierung erhältlich sind; Beispiele geeigneter cyclischer Monoolefine sind Cyclobuten, Cyclopenten, Cyclohexen, Cyclooncten, Cyclohepten, Norbonen oder 7- Oxanorbonen; Beispiele geeigneter acyclischer Monoolefine sind in Kohlenwasserstoffgemischen enthalten, die in der Erdölverarbeitung durch Cracken erhalten werden ($C_5$-Schnitt); Beispiele geeigneter, erfindungsgemäß zu verwendender oligomerer Polyole weisen eine Hydroxylzahl (OHZ) von 200 bis 450, ein zahlenmittleres Molekulargewicht Mn von 400 bis 1000 und ein massenmittleres Molekulargewicht $M_w$ von 600 bis 1100 auf;

Darüber hinaus enthalten die erfindungsgemäßen Beschichtungsmittel Vernetzer, welche mit den Hydroxylgruppen in den erfindungsgemäßen Verbindungen und in den Bindemitteln sowie gegebenenfalls in den Reaktiverdünnern Vernetzungsreaktionen eingehen.

Hierfür kommen die üblichen und bekannten Aminoplastharze in Betracht, deren Methylol- und/oder Methoximethylgruppen z. T. mittels Carbamat- oder Allophanatgruppen defunktionalisiert sein können. Vernetzer dieser Art werden in den Patentschriften US-A-4 710 542 und EP -B- 0 245 700 sowie in dem Artikel von B. Singh und Mitarbeiter "Carbamylmethylated Melamines, Novel Crosslinkers for the Coatings Industry" in Advanced Organic Coatings Science and Technology Series, 1991, Band 13, Seiten 193 bis 207, beschrieben.

Außer diesen Vernetzern können noch weitere Vernetzer vorhanden sein. Beispiele geeigneter weiterer Vernetzer sind Siloxangruppen enthaltende Verbindungen oder Harze, Anhydridgruppen enthaltende Verbindungen oder Harze, Epoxidgruppen enthaltende Verbindungen oder Harze, blockierte und/oder unblockierte, monomere und/oder oligomere Polyisocyanate und/oder Tris(alkoxycarbonylamino)triazine wie sie in den Patentschriften US-A-4 939 213,US-A-5 084 541, US-A-5 288 865 oder EP-A-0 604 922 beschrieben werden.

Die Viskosität der Vernetzer liegt im allgemeinen zwischen 10 und 20.000 mPas. Ihre Funktionalität liegt üblicherweise zwischen 1 und 5, insbesondere 1,5 und 4,5.

Je nach der Reaktivität des weiteren Vernetzers kann er den erfindungsgemäßen Beschichtungsmitteln direkt zugesetzt werden, wodurch ein sogenanntes Einkomponentensystem resultiert. Handelt es sich indes um einen besonders reaktiven Vernetzer, wie ein Polyisocyanat oder ein Epoxid, wird dieser im allgemeinen erst kurz vor der Verwendung den erfindungsgemäßen Beschichtungsmitteln zugesetzt. Hierbei resultiert ein sogenanntes Zwei- oder Mehrkomponentensystem, wie sie vor allem vom Autoreparatursektor her bekannt sind.

Die erfindungsgemäßen Beschichtungsmittel können übliche und bekannte Zusatzstoffe in üblichen und bekannten, wirksamen Mengen enthalten.

Beispiele geeigneter Zusatzstoffe sind Polymere, Vernetzer, Katalysatoren für die Vernetzung, Initiatoren, insbesondere Photoinitiatoren, Pigmente, Farbstoffe, Füllstoffe, Verstärkerfüllstoffe, Rheologiehilfsmittel, Lösemittel, Netz- und Dispergiermittel, Entschäumer, Haftvermittler, Additive zur Verbesserung der Untergrundbenetzung, Additive zur Verbesserung der Oberflächenglätte, Mattierungsmittel, Verlaufmittel, Filmbildehilfsmittel, Trockenstoffe, Hautverhinderungsmittel, Lichtschutzmittel, Korrosionsinhibitoren, Biozide, Flammschutzmittel, Polymerisationsinhibitoren, insbesondere Photoinhibitoren, oder Weichmacher, wie sie beispielsweise auf dem Kunststoff- oder Lacksektor üblich und bekannt sind.

Die Auswahl der Zusatzstoffe richtet sich nach dem gewünschten Eigenschaftsprofil der erfindungsgemäßen Beschichtungsmittel, Klebstoffe und Dichtungsmassen sowie deren speziellen Verwendungszwecken und kann daher vom Fachmann in einfacher Weise, gegebenenfalls unter Zuhilfenahme einfacher Vorversuche, getroffen werden.

**Beispiele**

**Beispiel 1**

**Herstellung einer erfindungsgemäßen Verbindung**

In einem 41-Stahlreaktor wurden 128 g Homopentaerythrit und 462 g Hexahydrophthalsäureanhydrid vorgelegt und während vier Stunden auf 120 °C erhitzt. Anschließend gab man 729,6 g Versatic$^{R}$-Säureglycidylester hinzu und erhitzte so lange, bis eine Säurezahl von 6,6 erreicht wurde. Die resultierende erfindungsgemäße Verbindung wurde bei 90 °C mit Butylacetat angelöst, so daß ein Festkörpergehalt von 85 Gew.-% resultierte. Das zahlenmittlere Molekulargewicht Mn der erfindungsgemäßen Verbindung betrug 1430, das massenmittlere Molekulargewicht Mw 1865.

Zum Vergleich der Viskositätswerte wurde eine Verdünnungskurve erstellt. Hierzu wurde mit Butylacetat der Festkörpergehalt der Originalprobe stufenweise erniedrigt, und die Viskosität der resultierenden Lösungen wurde mit einem Platte-Kegelviskosimeter gemessen. Die erhaltenen Ergebnisse finden sich in der Tabelle 1

Tabelle 1

| **Die Viskositäten der erfindungsgemäßen Verbindung bei verschiedenen Konzentrationen** | |
|---|---|
| Festkörpergehalt (Gew.-%) | Viskosität (dPa.s) |
| 85 | 131,2 |
| 80,4 | 27,2 |
| 74,8 | 7,7 |
| 69,6 | 2,3 |
| 64,4 | 0,9 |

**Vergleichsversuch V1**

**Herstellung einer nicht erfindungsgemäßen hyperverzweigten Verbindung V1**

In einem 4 1-Stahlreaktor wurden 134 g Trimethylolpropan und 462 g Hexahydrophthalsäureanhydrid vorgelegt und während 6,5 Stunden auf 120 °C erhitzt. Anschließend gab mal 684 g Versatic$^{R}$-Säureglycidylester hinzu und erhitzte so lange, bis eine Säurezahl von 4,5 erreicht wurde. Die resultierende hyperverzweigte Verbindung V1 wurde bei 90 °C mit Butylacetat angelöst, sodaß ein Festkörpergehalt von 84,4 Gew.-% resultierte. Das zahlenmittlere Molekulargewicht Mn der hyperverzweigten Verbindung V1 betrug 1499, das massenmittlere Molekulargewicht Mw 2485.

Zum Vergleich der Viskositätswerte wurde eine Verdünnungskurve erstellt. Hierzu wurde mit Butylacetat der Festkörpergehalt der Originalprobe erniedrigt, und die Viskosität der resultierenden Lösungen wurde mit einem Platte-Kegelviskosimeter gemessen. Die erhaltenen Ergebnisse finden sich in der Tabelle 2.

Tabelle 2

| Die Viskositäten der hyperverzweigten Verbindung V1 bei verschiedenen Konzentrationen | |
|---|---|
| Festkörpergehalt (Gew.-%) | Viskosität (dPa.s) |
| 78,7 | 33,6 |
| 75,5 | 9 |
| 70,0 | 3,4 |
| 64,8 | 1,2 |
| 60,0 | 0,4 |

**Vergleichsversuch V2**

**Herstellung einer nicht erfindungsgemäßen hyperverzweigten Verbindung V2**

In einem 41- Stahlreaktor wurden 136 g Pentaerythrit und 616 g Hexahydrophthalsäureanhydrid vorgelegt und während 6,5 Stunden auf 150 °C erhitzt. Anschließend gab man 904 g Versatic$^{R}$-Säureglycidylester hinzu und erhitzte so lange, bis eine Säurezahl von 0,7 erreicht wurde. Die resultierende hyperverzweigte Verbindung V2 wurde bei 90 °C mit Butylacetat angelöst, so daß ein Festkörpergehalt von 83,2 Gew.-% resultierte. Das zahlenmittlere Molekulargewicht Mn der hyperverzweigten Verbindung V2 betrug 1684, das massenmittlere Molekulargewicht Mw 3280.

Zum Vergleich der Viskositätswerte wurde eine Verdünnungskurve erstellt. Hierzu wurde mit Butylacetat der Festkörpergehalt der Originalprobe erniedrigt, und die Viskosität der resultierenden Lösungen wurde mit einem Platte-Kegelviskosimeter gemessen. Die erhaltenen Ergebnisse finden sich in der Tabelle 3.

Tabelle 3

| Die Viskositäten der hyperverzweigten Verbindung V2 bei verschiedenen Konzentrationen | |
|---|---|
| Festkörpergehalt (Gew.-%) | Viskosität (dPa.s) |
| 83,2 | 192,0 |
| 80,4 | 66,4 |
| 75,2 | 16,4 |
| 70, 1 | 4,8 |
| 65, 0 | 1,7 |

Der Vergleich der Ergebnisse der Tabellen 1 bis 3 belegt, daß die erfindungsgemäße Verbindung ab einem Festkörpergehalt von über 75 Gew.-% deutlich niedrigere Viskositäten aufweist als die hyperverzweigten Verbindungen V1 und V2, was anwendungstechnisch einen besonderen Vorteil darstellt.

**Beispiel 2 und Vergleichsversuche V3 und V4**

**Der Einfluß der erfindungsgemäßen Verbindung gemäß Beispiel 1 und der hyperverzweigten Verbindungen V1 und V2 gemäß den Vergleichsversuchen V1 und V2 auf die Viskosität von Bindemittellösungen**

Es wurden Mischungen des Polyacrylats (Bindemittel), wie es in der deutschen Offenlegungsschrift DE-A-44 07 415 in dem Beispiel E1 beschrieben ist, und der erfindungsgemäßen Verbindung und den hyperverzweigten Verbindungen V1 und V2 jeweils im Verhältnis 1 : 1 hergestellt.

In Beispiel 2 wurden das Polyacrylat E1 und die erfindungsgemäße Verbindung gemäß Beispiel 1 miteinander vermischt.

Im Vergleichsversuch V3 wurden das Polyacrylat E1 mit der hyperverzweigten Verbindung V1 gemäß Vergleichsversuch V1 und im Vergleichsversuch V4 wurden das Polyacrylat E1 mit der hyperverzweigten Verbindung V2 gemäß Vergleichsversuch V2 miteinander vermischt.

Das besagte Polyacrylat enthielt die folgenden Monomeren in den angegebenen Mengen einpolymerisiert: Styrol (23 Gew.-%), n- Butylmethacrylat (6 Gew.-%), t-Butylcyclohexylacrylat (14 Gew.-%), Methylmethacrylat (21 Gew.-%) sowie eine Mischung aus, bezogen auf die Mischung, 25 Gew.-% 3-Hydroxy-n-propylmethacrylat und 75 Gew.-% 2-Hydroxy-n-propylmethacrylat (36 Gew.-%).

Die resultierenden Mischungen wurden mit Butylacetat auf unterschiedliche Festkörpergehalte eingestellt. Zum Vergleich wurden Lösungen des Polyacrylats E1 in Butylacetat mit unterschiedlichen Festkörpergehalten herangezogen. Die Viskositäten der resultierenden Lösungen wurden mit einem Platte-Kegelviskosimeter bestimmt. Die Versuchsergebnisse finden sich in der Tabelle 4.

Tabelle 4

| **Der Einfluß der erfindungsgemäßen Verbindung gemäß Beispiel 1 und der hyperverzweigten Verbindungen V1 und V2 gemäß den Vergleichsversuchen V1 und V2 auf die Viskosität von Bindemittellösungen** | | |
|---|---|---|
| | Festkörpergehalt (Gew.-%) | Viskosität (dPas) |
| Polyacrylat E1 | 63,2 | 65,6 |
| | 62,5 | 58,4 |
| Beispiel 2 | 71,3 | 54,4 |
| | 64,5 | 5,1 |
| Vergleichsversuch V3 | 70,1 | 34,0 |
| | 59,4 | 2,6 |
| Vergleichsversuch V4 | 71,6 | 76,8 |
| | 62,8 | 6,2 |

Der Vergleich zeigt, daß die erfindungsgemäße Verbindung bei hohen Festkörpergehalten eine vergleichsweise niedrige Viskosität bewirkt.

**Beispiel 3 und Vergleichsversuche V5 und V6**

**Herstellung eines erfindungsgemäßen Beschichtungsmittels aus der Mischung gemäß Beispiel 2 sowie von nicht erfindungsgemäßen Beschichtungsmitteln aus den Mischungen gemäß den Vergleichsversuchen V3 und V4**

Für die Herstellung des erfindungsgemäße Beschichtungsmittels (= Beispiel 3) wurden 42,5 Teile der Mischung gemäß Beispiel 2 (Festkörpergehalt: 71,3 Gew.-%) mit 18,45 Teilen Desmodur$^R$ VPLS 2025 (blockiertes Polyisocyanat auf der Basis von Hexamethylendiisocyanat der Firma Bayer AG), 0,1 Teilen einer 10 %igen Lösung von Dibutylzinndilaurat in Butylacetat und 2,05 Teilen Butylacetat versetzt. Die Viskosität des resultierenden Lacks lag bei 17,4 dPas.

Für den Vergleichsversuch V5 wurden 35,1 Teile der Mischung gemäß Vergleichsversuch V3 (Festkörpergehalt: 70,1 Gew.-%) mit 16,89 Teilen eine Mischung aus 15,11 Teilen Desmodur$^R$ VPLS 2025, 0,1 Teilen einer 10 %igen Lösung von Dibutylzinndilaurat in Butylacetat und 1,68 Teilen Butylacetat versetzt. Die Viskosität des resultierenden Lacks lag bei 13,0 dPas.

Für den Vergleichsversuch V6 wurden 35,8 Teile der Mischung gemäß Vergleichsversuch V4 (Festkörpergehalt: 71,6 Gew.-%) mit 15,72 Teilen einer Mischung aus 14,06 Teilen Desmodur$^R$ VPLS 2025, 0,1 Teilen einer 10 %igen Lösung von Dibutylzinndilaurat in Butylacetat und 1,56 Teilen Butylacetat versetzt. Die Viskosität des resultierenden Lacks lag bei 22,4 dPas.

Die Lacke des Beispiels 3 und der Vergleichsversuche V5 und V6 wurden auf Glastafeln aufgerakelt, so daß eine Naßfilmstärke von 150 mikrometer resultierte, und während jeweils einer Stunde bei 60 °C und 130 °C eingebrannt,. Die Härte der resultierenden Beschichtungen wurde mit Hilfe der Pendeldämpfungs-Prüfung nach DIN 53157 bestimmt. Die Ergebnisse finden sich in der Tabelle 5.

Tabelle 5

| **Die Pendeldämpfung der Beschichtungen gemäß Beispiel 3 und gemäß den Vergleichsversuchen V5 und V6** | |
|---|---|
| | Pendeldämpfung (s) |
| Beispiel 3 - Härtungstemperatur: | |
| 60 °C<br>130 °C | 93,8<br>197,4 |
| Vergleichsversuch V5 - Härtungstemperatur: | |
| 60 °C<br>130 °C | 50,4<br>187,6 |
| Vergleichsversuch V6 - Härtungstemperatur: | |
| 60 °C<br>130 °C | 88,2<br>200,2 |

Der Vergleich der jeweiligen Pendeldämpfungen zeigt, daß das erfindungsgemäße Beschichtungsmittel gemäß Beispiel 3 auf der Basis der erfindungsgemäßen Verbindung gemäß Beispiel 1 bei signifikant niedrigerer Viskosität eine erfindungsgemäße Beschichtung ergibt, welche die gleiche Härte aufweist, wie die Beschichtungen auf der Basis der nicht erfindungsgemäßen Beschichtungsmittel gemäß den Vergleichsversuchen V5 und V6.

## Patentansprüche

**1.** Hyperverzweigte Verbindungen mit einer tetrafunktionellen Zentralgruppe der allgemeine Formel I

$$\mathbf{C\ [-A_q\text{-}X\text{-}]_m[-A_r\text{-}X\text{-}]_n[-A_s\text{-}X\text{-}]_o[-A_t\text{-}X\text{-}]_p} \quad \text{(I),}$$

worin die Indices und die Variablen die folgende Bedeutung haben:

$$m+n+o+p=4;$$

mit

m = eine ganze Zahl von 1 bis 3
n, o und p = 0 oder eine ganze Zahl von 1 bis 3;

q, r, s und t = eine ganze Zahl von 1 bis 5, wobei q > r, s, t;

X = -O-, -S- oder -NH-;

A = $-CR_2-$; mit

R = -H, -F, -Cl, -Br, -CN, $-NO_2$, $C_1$-$C_3$-Alkyl- oder -Haloalkyl- oder $C_1$-$C_3$-Alkoxirest oder, für q, r, s und/oder t = mindestens 2, $C_2$-$C_4$-Alkandiyl- und/oder Oxaalkandiylrest, welcher 2 bis 5 Kohlenstoffatome und/oder ein Sauerstoffatom -O-, welches 3 bis 5 Kohlenstoffatome des Restes -A- cyclisch überbrückt.

**2.** Die hyperverzweigte Verbindung nach Anspruch 1, **dadurch gekennzeichnet, daß** die Zentralgruppe I über Abstandshaltergruppen mit jeweils einer reaktiven funktionellen Gruppe verbunden ist.

**3.** Die hyperverzweigte Verbindung nach Anspruch 1 oder 2, **dadurch gekennzeichnet, daß** es sich bei der reaktiven funktionellen Gruppe um eine Gruppe der allgemeinen Formen II handelt

$$\textbf{-X-B} \qquad \text{(II)},$$

worin die Indices und die Variablen die folgende Bedeutung haben:

X = -O-, -S- oder -NH-;

B = -H, -Z oder $-R^1-(-Z^1)_u$ mit a = 1 oder 2 und

Z = $-C(O)-NH_2$,
$-C(O)-NH-C(O)-NH_2$,
$-C(O)-OR^2$,
$-C(O)-NH-C(O)-OR^2$,
$-C(O)-CH_2-C(O)-R^2$
$-O-C(O)-CH_2-C(O)-R^2$
$-C(O)-CR^3 = CH_2$,

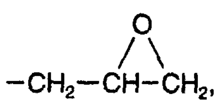

$-Si-(OR^4)_3$,
$-(-CH_2-)_v-CH = CH_2$ und $-C(O)-(-CH_2-)_v-CH = CH_2$; worin der Index v gleich 0 ist oder für eine ganze Zahl von 1 bis 3, insbesondere aber für 0 oder 1, steht;
$-C(O)-CH=CH-C(O)-O-R^2$,
$-C(O)-CH=CH-CH=CH-R^2$ und
$-C(O)-CH=CH-CH_2-CH=CH-R^2$;

$Z^1$ = -OH, $-NH_2$, -SH,
-COOH, - $SO_3H$, $-PO_3H$
$-O-C(O)-NH_2$,
$-O-C(O)-NH-C(O)-NH_2$,
-NCO;
$-NH-C(O)-OR^2$,
$-NH-C(O)-NH-C(O)-OR^2$,
$-O-C(O)-CH_2-C(O)-R^2$
$-O-C(O)-CH_2-C(O)-OR^2$
$-O-C(O)-CR^3 = CH_2$,

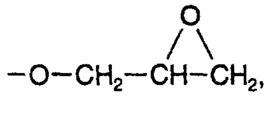

$-O-Si-(OR^4)_3$,
$-O-(-CH_2-)_v-CH = CH_2$ und
$-O-C(O)-(-CH_2-)_v-CH = CH_2$; worin der Index v für 0 steht oder für eine ganze Zahl von 1 bis 3, insbesondere aber für 0 und 1, steht;
$-O-C(O)-CH=CH-C(O)-O-R^2$,
$-O-C(O)-CH=CH-CH=CH-R^2$ und
$-O-C(O)-CH=CH-CH_2-CH=CH- R^2$; mit

$R^2$ = gegebenenfalls substituierter $C_1$-$C_{10}$-Alkyl-, $C_5$-$C_{10}$-Cycloalkyl-, $C_6$-$C_{20}$-Cycloalkylalkyl-, $C_6$-$C_{20}$-Alkylcycloalkyl-, $C_6$-$C_{10}$-Aryl-, $C_7$-$C_{20}$-Arylalkyl-, $C_7$-$C_{20}$-Alkylaryl-, $C_{11}$-$C_{26}$-Arylcycloalkyl- oder $C_{11}$-$C_{26}$-Cycloalkylarylrest, wobei die Alkylreste verzweigt oder unverzweigt sind;

$R^3$ = $C_1$-$C_4$-Alkylrest oder -CN; und

$R^4$ = gegebenenfalls substituierter $C_1$-$C_4$-Alkyl- oder Phenylrest;

$R^1$ = zwei- oder dreibindiger Rest, welcher sich

(i) von einem Alkan, Alken, Cycloalkan, Cycloalken, Alkylcycloalkan, Alkylcycloalken, Alkenyleycloalkan, oder Alkenylcycloalken, Aromaten und Heteroamaten sowie einem Alkyl-, Alkenyl-, Cycloalkyl-, Cycloalkenyl-, Alkylcyloalkyl-, Allcylcycloalkenyl-, Alkenylcycloalkyl- oder Alkenylcycloalkenyl-substitiuierten Aromaten oder Heteroaromaten ableiten; oder von

(ii) einem vorstehend genannten Rest , welcher mindestens ein Heteroatom in der Kette und/oder im Rest enthält, ableitetet; oder von

(iii) einem unter (i) oder (ii) genannten Rest, dessen Kette und/oder Ring substituiert ist ableitet.

**4.** Verwendung der hyperverzweigten Verbindung gemäß einem der Ansprüche 1 bis 3 als funktionelle Komponente in Mehrstoffmischungen oder für die Herstellung dendrimerer Verbindungen höherer Generationen.

**5.** Mehrstoffmischung, welche eine hyperverzweigte Verbindung gemäß einem der Ansprüche 1 bis 3 enthält.

**6.** Dendrimere Verbindung, welche eine hyperverzweigte Verbindung gemäß einem der Ansprüche 1 bis 3 als Zentralgruppe enthalten.

**7.** Die Mehrstoffmischung nach Anspruch 5, **dadurch gekennzeichnet, daß** es sich um Beschichtungsmittel, Klebstoffe oder Dichtungsmassen handelt.

**8.** Die Beschichtungsmittel, Klebstoffe und Dichtungsmassen nach Anspruch 7, **dadurch gekennzeichnet, daß** sie lufttrocknend, physikalisch trocknend, thermisch härtbar, mit aktinischem Licht härtbar oder mit Elektronenstrahlung härtbar sind.

**9.** Die Beschichtungsmittel nach Anspruch 7 oder 8, **dadurch gekennzeichnet, daß** es sich um in wäßrigen oder organischen Medien gelöste oder dispergierte Spritzlacke, flüssige Lacke, Pulverlacke oder Pulverslurries handelt.

**10.** Die Beschichtungsmittel nach einem der Ansprüche 7 bis 9, **dadurch gekennzeichnet, daß** es sich um Lacke für den Industriesektor, Möbellacke, Bautenanstrichmittel, Automobilserienlacke oder um Autoreparaturlacke handelt.

**Claims**

**1.** A hyperbranched compound having a tetrafunctional central group of the general formula I

$$\mathbf{C[-A_q-X-]_m[-A_r-X-]_n[-A_s-X-]_o[-A_t-X-]_p} \quad \text{(I),}$$

in which the indices and variables have the following meanings:

$$m + n + o + p = 4;$$

where

m = an integer from 1 to 3 and

n, o and p = 0 or an integer from 1 to 3;

q, r, s and t = an integer from 1 to 5, where q > r, s, t;

X = -O-, -S- or -NH-;

A = $-CR_2-$; where

R = -H, -F, -Cl, -Br, -CN, $-NO_2$, $C_1$-$C_3$ alkyl or $C_1$-$C_3$ haloalkyl or $C_1$-$C_3$ alkoxy radical or, if q, r, s and/or t = at least 2, a $C_2$-$C_4$ alkanediyl and/or $C_2$-$C_4$ oxaalkanediyl radical which cyclically bridges 2 to 5 carbon atoms, and/or an oxygen atom -O-, which cyclically bridges 3 to 5 carbon atoms, of the radical -A-.

**2.** The hyperbranched compound as claimed in claim 1, wherein the central group I is connected to one reactive functional group in each case via spacer groups.

**3.** The hyperbranched compound as claimed in claim 1 or 2, wherein the reactive functional group comprises a group of the general formula II

$$\mathbf{-X-B} \qquad (II)$$

in which the indices and variables have the following meanings:

X = -O-, -S- or -NH-;

B = -H, -Z or $-R^1-(-Z^1)_u$ where u = 1 or 2 and

Z = $-C(O)-NH_2$,
$-C(O)-NH-C(O)-NH_2$,
$-C(O)-OR^2$,
$-C(O)-NH-C(O)-OR^2$,
$-C(O)-CH_2-C(O)-R^2$
$-O-C(O)-CH_2-C(O)-R^2$
$-C(O)-CR^3 = CH_2$,

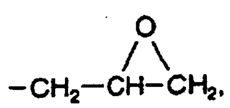

$-Si-(OR^4)_3$,
$-(-CH_2-)_v-CH = CH_2$ and
$-C(O)-(-CH_2-)_v-CH = CH_2$ ; in which the index v is 0 or is an integer from 1 to 3, but in particular 0 or 1;
$-C(O)-CH=CH-C(O)-O-R^2$,
$-C(O)-CH=CH-CH=CH-R^2$ and
$-C(O)-CH=CH-CH_2-CH=CH-R^2$;

$Z^1$ = -OH, $-NH_2$, -SH,
-COOH, $-SO_3H$, $-PO_3H$
$-O-C(O)-NH_2$,
$-O-C(O)-NH-C(O)-NH_2$,
-NCO;
$-NH-C(O)-OR^2$,
$-NH-C(O)-NH-C(O)-OR^2$,
$-O-C(O)-CH_2-C(O)-R^2$
$-O-C(O)-CH_2-C(O)-OR^2$
$-O-C(O)-CR^3 = CH_2$;

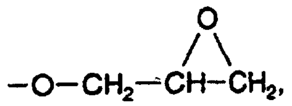

$-O-Si-(OR^4)_3$,
$-O-(-CH_2-)_v-CH = CH_2$ and
$-O-C(O)-(-CH_2-)_v-CH = CH_2$ ; in which the index v is 0 or an integer from 1 to 3, but in particular 0 and 1;
$-O-C(O)-CH=CH-C(O)-O-R^2$,
$-O-C(O)-CH=CH-CH=CH-R^2$ and
$-O-C(O)-CH=CH-CH_2-CH=CH-R^2$; where

$R^2$ = unsubstituted or substituted $C_1$-$C_{10}$ alkyl, $C_5$-$C_{10}$ cycloalkyl, $C_6$-$C_{20}$ cycloalkylalkyl, $C_6$-$C_{20}$ alkylcycloalkyl, $C_6$-$C_{10}$ aryl, $C_7$-$C_{20}$ arylalkyl, $C_7$-$C_{20}$ alkylaryl, $C_{11}$-$C_{26}$ arylcycloalkyl or $C_{11}$-$C_{26}$ cycloalkylaryl radical, the alkyl radicals being branched or unbranched;
$R^3$ = $C_1$-$C_4$ alkyl radical or -CN; and
$R^4$ = unsubstituted or substituted $C_1$-$C_4$ alkyl radical or phenyl radical;
$R^1$ = divalent or trivalent radical derived from

(i) an alkane, alkene, cycloalkane, cycloalkene, alkylcycloalkane, alkylcycloalkene, alkenylcycloalkane, or alkenylcycloalkene, aromatics and heteroaromatics, and also an alkyl-, alkenyl-, cycloalkyl-, cycloalkenyl-, alkylcycloalkyl-, alkylcycloalkenyl-, alkenylcycloalkyl- or alkenylcycloalkenyl- substituted aromatics or heteroaromatics; or

(ii) an abovementioned radical which contains at least one heteroatom in the chain and/or in the ring; or

(iii) a radical mentioned under (i) or (ii) whose chain and/or ring is substituted.

**4.** The use of a hyperbranched compound as claimed in any of claims 1 to 3 as a functional component in multisubstance mixtures or for preparing dendrimeric compounds of higher generations.

**5.** A multisubstance mixture which comprises a hyperbranched compound as claimed in any of claims 1 to 3.

**6.** A dendrimeric compound which comprises as central group a hyperbranched compound as claimed in any of claims 1 to 3.

**7.** The multisubstance mixture of claim 5, which is a coating composition, adhesive or sealing compound.

**8.** The coating composition, adhesive or sealing compound as claimed in claim 7, which is airdrying, physically drying, thermally curable, curable with actinic light or curable with electron beams.

**9.** The coating composition as claimed in claim 7 or 8, which comprises - dissolved or dispersed in aqueous or organic media - a spray coating material, liquid coating material, powder coating material or powder slurry.

**10.** The coating composition as claimed in any of claims 7 to 9, which is a coating material for the industrial sector or a furniture coating, architectural coating, automotive OEM coating, or automotive refinish coating material.

## Revendications

**1.** Composés hyper-ramifiés ayant un groupe central tétrafonctionnel de formule générale I

$$C[-A_q-X-]_m[-A_r-X-]_n[-A_s-X-]_o[-A_t-X-]_p \qquad (I),$$

dans laquelle les indices et les variables prennent la signification suivante :

$$m + n + o + p = 4 ;$$

avec

m = un nombre entier de 1 à 3
n, o et p = 0 ou un nombre entier de 1 à 3 ;

q, r, s et t = un nombre entier de 1 à 5, où q > r, s, t ;

X = -O-, -S- ou -NH- ;
A = -CR2- ; avec
R = -H, -F, -Cl, -Br, -CN, -NO2, un résidu alkyle ou halogénoalkyle en C1 à C3 ou un résidu alcoxy en C1 à C3 ou, pour q, r, s et/ou t = au moins 2, un résidu alcanediyle et/ou oxa-alcane-diyle en C2 à C4, lequel jette un pont cyclique sur 2 à 5 atomes de carbone et/ou un atome d'oxygène -O-, lequel jette un pont cyclique sur 3 à 5 atomes de carbone du résidu -A-.

**2.** Composé hyper-ramifié selon la revendication 1, **caractérisé en ce que** le groupe central I est lié, à chaque fois, à un groupe fonctionnel réactif par l'intermédiaire de groupes espaceurs.

**3.** Composé hyper-ramifié selon la revendication 1 ou 2, **caractérisé en ce que**, quant au groupe fonctionnel réactif, il s'agit d'un groupe de formule générale II

-X-B (II),

dans laquelle les indices et les variables prennent la signification suivante :

X = -O-, -S- ou -NH- ;
B = -H, -Z ou -R1-(-Z1)u avec u = 1 ou 2 et
Z = -C(O)-NH2,
-C(O)-NH-C(O)-NH2,
-C(O)-OR2,
-C(O)-NH-C(O)-OR2,
-C(O)-CH2-C(O)-R2,
-O-C(O)-CH2-C(O)-R2,
-C(O)-CR3=CH2,

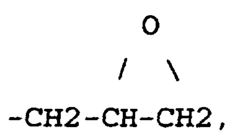

-Si-(OR4)3,
-(-CH2-)ν-CH=CH2 et
-C(O)-(-CH2-)ν-CH=CH2 ; dans lesquels l'indice ν est égal à 0 ou bien représente un nombre entier de 1 à 3, mais en particulier représente 0 ou 1 ;
-C(O)-CH=CH-C(O)-O-R2,
-C(O)-CH=CH-CH=CH-R2, et
-C(O)-CH=CH-CH2-CH=CH-R2 ;
Z1 = -OH, -NH2, -SH,
-COOH, -SO3H, -PO3H,
-O-C(O)-NH2,
-O-C(O)-NH-C(O)-NH2,
-NCO ;
-NH-C(O)-OR2,

-NH-C(O)-NH-C(O)-OR2,
-O-C(O)-CH2-C(O)-R2,
-O-C(O)-CH2-C(O)-OR2,
-O-C(O)-CR3=CH2,

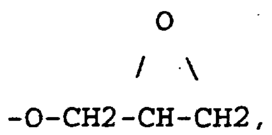

-O-Si-(OR4)3,
-O-(-CH2-)ν-CH=CH2 et
-O-C(O)-(-CH2-)ν-CH=CH2 ; dans lesquels l'indice ν représente 0 ou représente un nombre entier de 1 à 3, mais en particulier représente 0 et 1 ;
-O-C(O)-CH=CH-C(O)-O-R2,
-O-C(O)-CH=CH-CH=CH-R2, et
-O-C(O)-CH=CH-CH2-CH=CH-R2; avec

R2 = un résidu alkyle en C1 à C10, cycloalkyle en C5 à C10, cycloalkylalkyle en C6 à C20, alkylcycloalkyle en C6 à C20, aryle en C6 à C10, arylalkyle en C7 à C20, alkylaryle en C7 à C20, arylcycloalkyle en C11 à C26 ou cycloalkylaryle en C11 à C26, éventuellement substitué, les résidus alkyle étant ramifiés ou non ramifiés ;
R3 = un résidu alkyle en C1 à C4, ou -CN ; et
R4 = un résidu alkyle en C1 à C4, ou phényle éventuellement substitué ;
R1 = un résidu bivalent ou trivalent, qui est dérivé de

(i) un alcane, alcène, cycloalcane, cycloalcène, alkylcycloalcane, alkylcycloalcène, alcénylcycloalcane, ou alcénylcycloalcène, une substance aromatique et hétéroaromatique, ainsi qu'une substance aromatique ou hétéroaromatique à substitution alkyle, alcényle, cycloalkyle, cycloalcényle, alkylcycloalkyle, alkylcycloalcényle, alcénylcycloalkyle, ou alcénylcycloalcényle ; ou de
(ii) un résidu cité précédemment, qui contient au moins un hétéroatome dans la chaîne et/ou dans le résidu ; ou de
(iii) un résidu cité sous (i) ou (ii), dont la chaîne et/ou le cycle est substitué(e).

4. Utilisation du composé hyper-ramifié selon l'une quelconque des revendications 1 à 3, en tant que composant fonctionnel dans des mélanges de plusieurs matières ou pour la préparation de composés dendrimères de générations plus élevées.

5. Mélange de plusieurs matières, qui contient un composé hyper-ramifié selon l'une quelconque des revendications 2 à 3.

6. Composé dendrimère, qui contiennent un composé hyper-ramifié selon l'une quelconque des revendications 1 à 3 en tant que groupe central.

7. Mélange de plusieurs matières selon la revendication 5, **caractérisé en ce qu'**il s'agit d'agents de revêtement, d'adhésifs, ou de masses d'étanchéité.

8. Agents de revêtement, adhésifs et masses d'étanchéité selon la revendication 7, **caractérisés en ce qu'**ils sont durcissables par séchage à l'air, par séchage physique, thermodurcissables, durcissables à la lumière actinique ou durcissables par irradiation d'électrons.

9. Agents de revêtement selon la revendication 7 ou 8, **caractérisés en ce qu'**il s'agit de peintures à appliquer au pistolet, de peintures liquides, de peintures en poudre ou de suspensions de poudre, dissoutes ou dispersées dans des milieux aqueux ou organiques.

10. Agents de revêtement selon l'une quelconque des revendications 7 à 9, **caractérisés en ce qu'**il s'agit de peintures pour le secteur industriel, de peintures pour meubles, d'enduits pour le bâtiment, de peintures de série pour automobiles, ou de peintures pour la réparation automobile.